Europäisches Patentamt

European Patent Office

Office européen des brevets

(11) Publication number: **0 271 277**
**A2**

(12) # EUROPEAN PATENT APPLICATION

(21) Application number: **87310602.5**

(22) Date of filing: **02.12.87**

(51) Int. Cl.⁴: **G02B 6/44**

(30) Priority: **12.12.86 GB 8629707**

(43) Date of publication of application:
**15.06.88 Bulletin 88/24**

(84) Designated Contracting States:
**AT BE CH DE FR IT LI NL SE**

(71) Applicant: **Telephone Cables Limited**
**Chequers Lane**
**Dagenham Essex RM9 6QA(GB)**

(72) Inventor: **George, Edward Kitchener**
**93 Nelson Road**
**South Hornchurch Essex(GB)**
Inventor: **Jung, Roger Erwin**
**38 Tufton Road**
**Chingford London E4 8LE(GB)**
Inventor: **Mills, Brian Peter**
**7 Candover Road**
**Hornchurch Essex RM12 4TY(GB)**

(74) Representative: **Kirby, Harold Victor Albert**
**The General Electric Company, p.l.c. Central**
**Patent Department Wembley Office GEC**
**Research Centre East Lane**
**Wembley Middlesex HA9 7PP(GB)**

(54) **Distribution frame.**

(57) A distribution frame for an optical fibre transmission system comprises connection fibres which pass between pairs of resilient separating straps (3) which bear against the sides of the connection fibres and hold them apart.

*Fig.1.*

## Distribution Frame.

This invention relates to distribution frames for optical fibre transmission systems, such distribution frames being arranged to accommodate connections between optical fibre cables and individual optical fibres, hereinafter referred to as connection fibres.

These distribution frames may be required to provide connections between a plurality of optical cables and a respective multiplicity of connection fibres which may be in the form of single or multifibre elements or cords, having the fibre or fibres protected by an outer coating or cover. It is an object of this invention to provide a means whereby optical fibre cords may be readily introduced into the frame and to provide a support for the units, without risk of damage to the optical fibres within them.

According to the invention, a distribution frame for an optical fibre transmission system is provided in which connection fibres, as hereinbefore defined, pass between pairs of resilient separating straps which bear against the sides of the connection fibres and hold them apart.

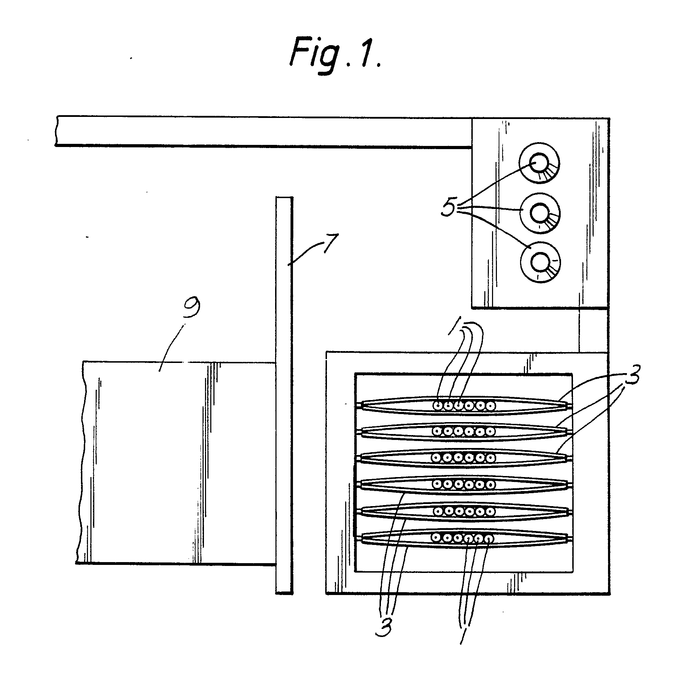

One distribution frame in accordance with the invention will now be described by way of example with reference to Figure 1, the accompanying - schematic drawing which represents a perspective view of part of the top of the frame.

The distribution frame is in the form of a cabinet having a structural framework consisting of a rectangular base with a support post extending upwards from each corner. To the two support posts at each end of the framework there are attached three sheet metal outer panels. Two inner panels 7, extending the full height of the frame, are disposed between and parallel to the outer panels. A number of organiser trays 9, for accommodating the ends of optical fibres of optical fibre cables entering the cabinet through cable entry sleeves 5, are supported between the inner panels 7.

These optical fibres are connected in any convenient manner, for example by couplers supported by the inner panels, to outgoing fibres in the form of a plurality of cords, each comprising single or multiple fibres within a protective coating or cover. The cords 1 pass from the spaces defined by the inner and side panels between pairs of resilient separating straps 3, for example of rubber or like material, which engage the sides of the cords 1 and hold them apart whilst serving to support them.

## Claims

1. A distribution frame for an optical fibre transmission system in which connection fibres, as hereinbefore defined, pass between pairs of resilient separating straps (3) which bear against the sides of the connection fibres and hold them apart.

# Fig .1.